Europäisches Patentamt

European Patent Office

Office européen des brevets

(11) Publication number: **0 181 075**
**B1**

(12) **EUROPEAN PATENT SPECIFICATION**

(45) Date of publication of patent specification: **02.11.88**

(51) Int. Cl.⁴: **C 12 M 1/22**

(21) Application number: **85306742.9**

(22) Date of filing: **23.09.85**

(54) **Petri dish for cultivating bacteria and a method of testing drug susceptibility.**

(30) Priority: **09.10.84 JP 212163/84**

(43) Date of publication of application:
**14.05.86 Bulletin 86/20**

(45) Publication of the grant of the patent:
**02.11.88 Bulletin 88/44**

(84) Designated Contracting States:
**CH DE FR GB IT LI**

(56) References cited:
**DE-A-3 102 571**
**DE-A-3 218 532**
**DE-C- 839 245**
**FR-A-2 514 366**
**US-A-2 677 646**
**US-A-2 874 091**
**US-A-3 073 750**

(73) Proprietor: **KOBAYASHI PHARMACEUTICAL CO. LTD.**
**25, Doshomachi 5-chome**
**Higashi-ku Osaka (JP)**

(72) Inventor: **Asano, Kenzi**
**2-7-30-603, Sakasedai Takarazuka-shi**
**Hyogo (JP)**
Inventor: **Sugahara, Kazuyuki**
**Ichiba Bldg. 204 15-10, Wakakusamachi**
**Nagasaki-shi Nagasaki (JP)**
Inventor: **Takakura, Tetsuya**
**Haitsu Koho 116 3-12-8, Hattorinishimachi**
**Toyonaka-shi Osaka (JP)**

(74) Representative: **Barker, Rosemary Anne et al**
**Barlow, Gillett & Percival 94 Market Street**
**Manchester M1 1PJ (GB)**

Courier Press, Leamington Spa, England.

# 0 181 075

**Description**

This invention relates to petri dishes for cultivating bacteria. The dishes may be used for such purposes as isolation of bacteria, enrichment culture, and drug susceptibility testing. The invention also relates to a method of testing drug susceptibility of bacteria which may be isolated from a clinical specimen etc. or purely cultured after their isolation, using the Petri dish of the invention.

When testing bacteria in a hospital, in order to determine the pathogen to which an infectious disease is ascribed, it is necessary to perform operations such as the isolation culture and the enrichment culture of bacteria from the material to be investigated. It can be difficult to identify pathogenic bacteria from the clinical diagnosis due to complication of the pathologic characteristics of the infectious disease. Results obtained by testing bacteria have come to be regarded as important.

The media suitable for the growth of the bacteria are varied, depending upon the kind of bacteria, however. So when screening pathogenic bacteria, a preparation of the medium adequate each of the possible bacteria is needed. The bacteria which may be found in a blood specimen are specified to be *staphylococcus, streptococcus, pneumococcus, enterococcus, haemophylus, salmonella, escherichia coli, pseudomonas aeruginosa, anaerobic bacterium, campylobacter, brucella,* etc. and usually in order to isolate any of these bacteria, there is a necessity of preparing each of blood agar medium, chocolate agar medium, isolation medium for *anaerobic bacterium,* medium for enrichment, etc. all for each single specimen. Furthermore, *salmonella, dysentery bacillus, pathogenic coli, yersinia enterocolitica, klebsiella oxytoca, vibrio, staphylococcus, bacillus cereus,* etc. may be detected from the feces and as medium for any of these bacteria, a preparation of each of BTB lactose agar medium, DHL agar medium, SS agar medium, isolation medium for *anaerobic bacterium,* medium for enrichment, etc. is needed.

When using the conventional petri dish for cultivating bacteria, however, it is required to heat the solution for a long time, because the medium contains agar as well as to put the Petri dish horizontally in a stationary manner during the coagulation thereof, resulting in a complicated operation. In addition, almost all the conventional petri dishes for cultivating bacteria have one medium per dish, so the number of Petri dishes and inoculation operations for one specimen becomes considerable. Although a method may be conceived in which several kinds of media are poured separately into sub-sections of a Petri dish the inside of which is subdivided with partitions, it is still necessary to perform individual inoculation operations because there are differences in level between media and between the media and the partitions of the Petri dish.

In addition to the necessity of complicated operations such as solution, sterilization, separate pouring, and coagulation of the component of the media, each operation has to be performed as aseptically as possible. Usually, a separate pouring of one medium per one Petri dish increases the number of Petri dishes necessary for one specimen, so it is substantially impossible to prepare adequately the necessary amount of media in the hospital's test room where the number of specimens and the kind of bacteria being handled are not constant. For this reason, it is common to make or purchase the estimated amount necessary for a predetermined period and consume these gradually while storing the stock of unused dishes in a refrigerator. The dishes have a lid simply fitting around the container for the media, so there is a problem of humidity and various bacteria contaminating the media.

Before using conventional media, a drying operation is indispensable, but media which are in a deaired state during storage or at the time of being purchased, dissolve oxygen from the air during such a drying operation. So, if *anaerobic bacteria* are handled, the filled dishes need to be placed in an anaerobic chamber after drying.

Next, when applying chemotherapy to a bacterial infectious disease, it is desirable to select and use the drug most effective to the bacterium causing such a disease. Nevertheless, the kind and concentration of the drug effective to the bacteria depend on the kind of the bacteria and the recent spread of the chemotherapy triggers an appearance of bacterial stock (resistant bacteria) which show a tolerance to a drug which was previously effective so that if any bacterium causing the infectious disease is determined, it is substantially impossible to assume that a drug will be effective. For this reason, for clinically adequate and reasonable therapy, it ought not to be forgotten that the drug susceptibility test is one of the important items among the bacterial items to be investigated.

As a method of testing the drug susceptibility of the bacteria, dilution methods, diffusion methods, and a nephelometry method are available for use. An agar plate dilution method using agar medium and a broth dilution method are both dilution methods. A sensitive tablet method, a sensitivity disk method, a decantation plate method, and a vertical diffusion method are diffusion methods.

The agar plate dilution method and the sensitivity disk method are the chief methods used.

Although the sensitivity disk method, being easy to operate and capable of testing a plurality of drugs and concentration, simply by one Petri dish, is widely utilized, the thickness, inclination, or kind of medium on which the disk is put can easily make a fluctuation in the magnitude of the inhibition circle which occurs. The test is thus apt to lack reproducibility. A 3-concentration disk method in which a judgement is made on the basis not of the magnitude of the inhibition circle but only of its existence, is sometimes used. However, the setup width of the drug concentration is larger, so such a method is not suitable for measuring minimum inhibitory concentration (MIC) of the bacteria to be tested.

The agar plate dilution method uses media which have a drug of a predetermined concentration

2

incorporated therein in advance. This is better is reproducibility and can freely set the concentration of the drug. Since it needs one medium per one drug or concentration setup, having to perform the inoculation operation for every one medium, it is complicated.

The conventional drug susceptibility test, the purpose of which is to decide a method of treatment, regards the drug concentration at which the growth of all the bacteria is inhibited as important, but does not pay attention to the magnitude of the drug susceptibility—of the stock of bacteria to be tested—at each drug concentration. However, in order to get more information about the infectious disease, it is desirable to obtain further detailed information about the bacteria causing such a disease.

The present invention intends to solve the problems possessed by the foregoing conventional petri dish for cultivating the bacteria, i.e. the forced necessity of executing complicated operations in order to prepare the media. Forms of the invention address the difficulty that the inoculation operation can only inoculate the bacteria into one medium in one operation.

A further aim of the present invention is to solve the problem that the media get dry or are contaminated by humidity and various bacteria during storage and in transit.

A still further aim of forms of the present invention is to solve the problems of the complicated operations needed for the media preparation and the inoculation in the above-mentioned conventional test method while avoiding the lower reproducibility of the sensitivity disk method.

For that purpose, the present invention provides a Petri dish for cultivating bacteria characterised in that the mouth at the upper edge of the Petri dish proper is covered with at least one sheet and at least one small aperture for filling the dish is provided in the bottom of said Petri dish.

The Petri dish of the invention is filled by pouring in through the small aperture in the bottom of the dish while the dish is inverted. The small aperture may be provided with a detachable sealing lid.

The sheet may be a sheet (hereinafter referred to as the pervious sheet) pervious to the component(s) of the medium or media with which the Petri dish is filled and may be able to hold the bacteria. It is also possible for the sheet to be a sheet (hereinafter referred to as the impervious sheet) impervious to the component of the media, in which case the sheet needs to be able to cover and be detachable from the Petri dish proper. The impervious sheet may also be impervious to water and oxygen. It is further acceptable to cover the outside of a pervious sheet with a detachable impervious sheet.

One form of Petri dish in accordance with this invention is subdivided by partitions, the height of which extends between the bottom of said Petri dish and the said sheet which covers the mouth of the dish.

In a second aspect, the invention provides a method of testing drug susceptibility, utilising this sub-divided (i.e. fractionized) form of dish.

The method of the present invention, using the fractionized Petri dish with a pervious sheet as the cultivation surface, separates media which are different in kind from each other. The media may differ from each other in the kind of drug which they contain or the media may contain drugs which are of the same in kind, but differ in the concentration from each other in each fraction, into which the bacteria are inoculated, thereby solving the problems of the above-mentioned conventional methods.

Preparation of such a Petri dish can be easily and aseptically performed by pouring in each of its media, separately from the others, through the small aperture(s) provided on the bottom of the Petri dish. When performing the inoculation operation, the unification of the height of the cultivation surface which is accomplished by the sheet covering the mouth of the dish, enables the entire region of the fractionized medium to be inoculated with the bacteria in one operation.

When using an impervious sheet, the medium does not get dry. Humidity and various bacteria do not contaminate the medium during storage or in transit. Since the medium is sealed from direct contact with the air, its deterioration due to oxidation is inhibited. Also at the time of the inoculation operation, removal of the impervious sheet exposes a cultivation surface which is all in the same plane even if the medium is fractionized. This enables inoculating bacteria into the entire region of such a fractionized medium in one operation.

The invention will be described further, by way of example, with reference to the accompanying drawings in which:—

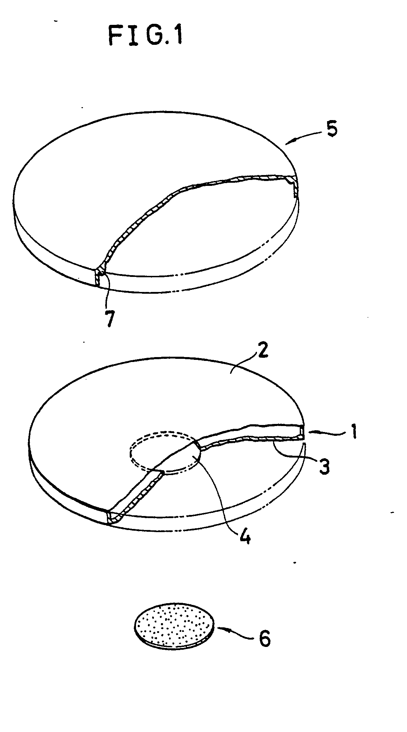

Figure 1 is a partially cut-away exploded perspective view of 1st embodiment, using a pervious sheet, of the Petri dish for cultivating the bacteria according to the present invention;

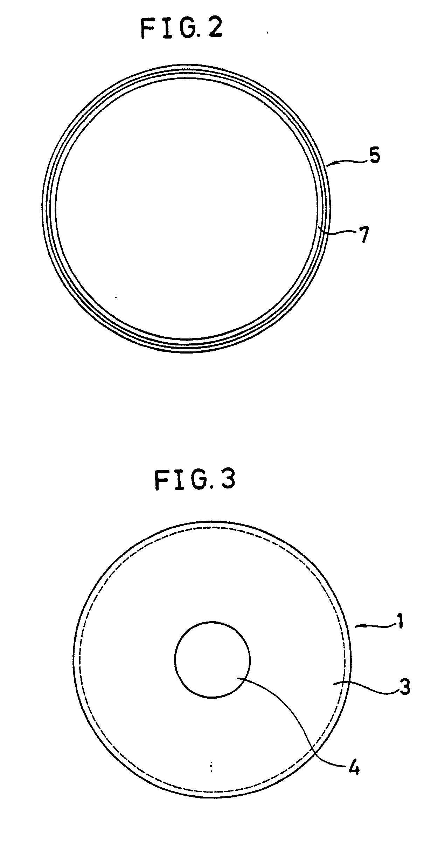

Figure 2 is a bottom view of the Petri dish lid of 1st embodiment;

Figure 3 is a bottom view of the Petri dish proper of 1st embodiment;

Figure 4 is a partially cut-away exploded perspective view of 2nd embodiment;

Figure 5 is a bottom view of the Petri dish lid of 2nd embodiment;

Figure 6 is a bottom view of the Petri dish proper of 2nd embodiment;

Figure 7 is a bottom view of the Petri dish proper of 3rd embodiment using an impervious sheet;

Figure 8 is a sectional view taken on the line A—A of Figure 7;

Figure 9 is a bottom view of the Petri dish proper of 4th embodiment;

Figure 10 is a sectional view taken on the line B—B of Figure 9;

Figure 11 is a partially cut-away exploded perspective view of 5th embodiment;

Figure 12 is a partially cut-away exploded perspective view of 6th embodiment using both of a pervious sheet and an impervious sheet;

Figure 13 is a bottom view of the Petri dish proper of 6th embodiment;

# 0 181 075

Figure 14 is a sectional view taken on the line C—C of Figure 13;

Figure 15 is a partially cut-away exploded perspective view of 7th embodiment.

(1) is the Petri dish proper, made of plastic or glass and being preferably transparent. The mouth at the upper edge of the dish is covered with a pervious sheet (2). The bottom (3) of the Petri dish is provided with a small aperture (4). The above-mentioned pervious sheet (2) needs to possess holes or pores through which component(s) of the medium filled between the Petri dish proper (1) and the pervious sheet (2) can percolate to the surface of the previous sheet (2). The material of the pervious sheet may be a porous film or a porous rubber film. Preferred are a membrane of synthetic resin such as cellulose ester, polypropylene, polycarbonate, polyvinylidene fluoride, or aromatic polymer. In the case where the medium with which the Petri dish is filled contains solidifying agent such as agar, materials such as cloth and paper which have wider pores may be applicable. If a synthetic resin film which is hydrophobic is used, a hydrophilic treatment should be applied to it in advance. As for the strength of the pervious sheet (2), the requirement is to achieve a flat surface of the medium, when the Petri dish is filled therewith. If the material needs any reinforcement, synthetic fiber, etc. can act in such a role. In the case of a solid medium, it can be sufficient to insert a flat reinforcing member into the clearance between the pervious sheet (2) and a Petri dish lid (5) which is referred to below, until the medium has solidified. Furthermore, the small aperture (4) on the bottom (3) of the Petri dish preferably needs to be provided with a detachable sealing lid (6). Necessity for this is sometimes reduced almost to nil in the case where the medium with which the Petri dish is filled contains a solidifying agent such as agar. Sealing the aperture (6) preserves a sterile state of the interior of the Petri dish (1).

(5) is a Petri dish lid, made of the plastic or glass and preferably transparent. The internal periphery of this is equipped with an annular projection (7). This projection (7) is provided to form a clearance between the pervious sheet (2) and the Petri dish lid (5), when the Petri dish lid (5) is fitted over the Petri dish proper (1), whereby a colony of the bacteria which is grown on the surface of the pervious sheet (2) is prevented from sticking to the internal face of the Petri dish lid (5). The projection (7) also serves to keep adequate airtighness with regard to the outside. The position of the above-mentioned projection (7) is not necessarily limited to the internal periphery of the Petri dish lid (5). It may acceptably be located on the periphery of the pervious sheet (2) or the periphery at the upper edge of the Petri dish proper (1).

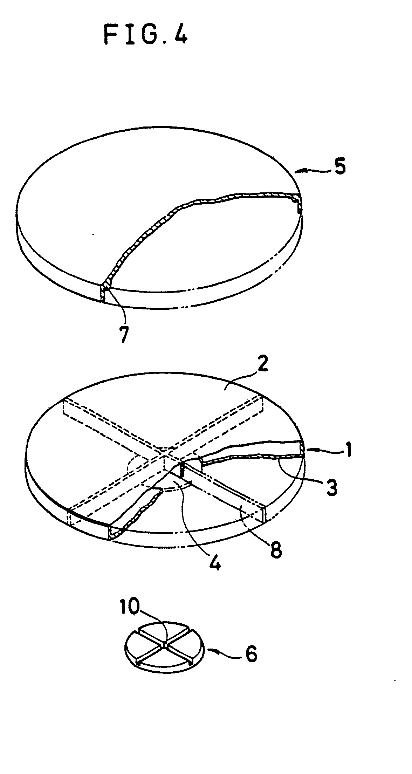

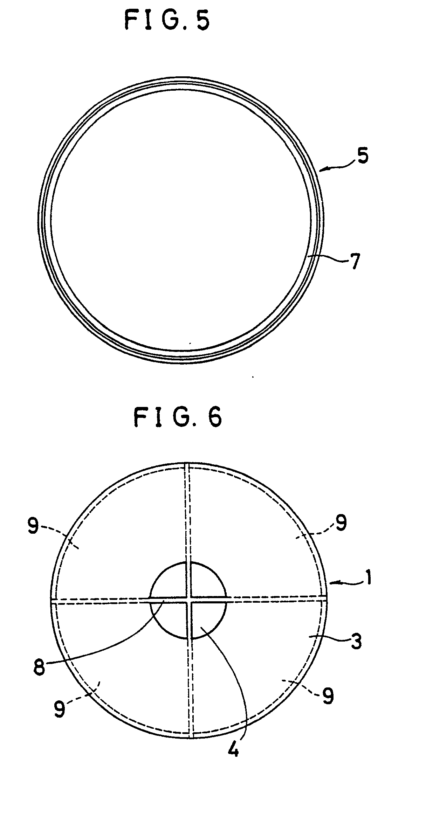

As shown in Figures 4 to 6 the above-mentioned Petri dish proper (1) can be provided with partitions (8) the height of which matches the distance between the bottom (3) of the Petri dish and the pervious sheet (2). In that case, all the sub-sections (9) of the dish created by the partitions (8) are required to be accessible through one or more small apertures (4). Figures 4 to 6, for example, show a dish provided with four sub-sections, and a small aperture (4) at the center of the bottom (3) of the Petri dish, at which the partitions (8) intersect, to let the small aperture (4) communicate with each of the sub-sections (9), in addition to which the sealing lid (6) may be a plug possessing a cross slit (10) as shown.

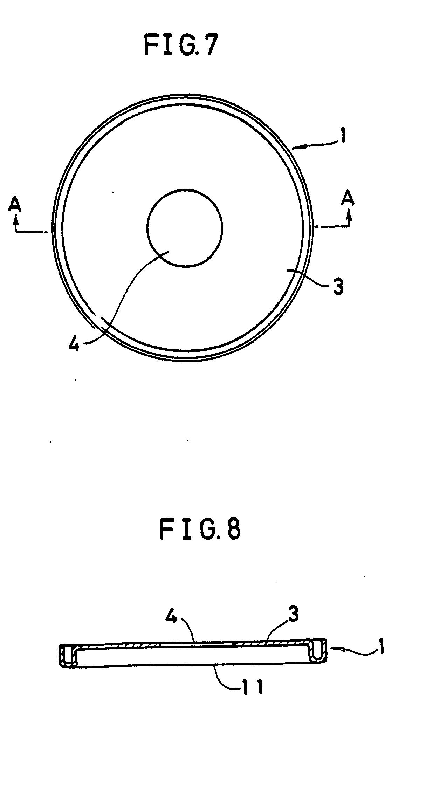

Referring next to Figure 7 to 11, illustrating an embodiment using an impervious sheet, the mouth at the upper edge of the Petri dish proper (1) is detachably covered with the impervious sheet (11). The impervious sheet (11) may have any thickness and is required to be impervious to water and oxygen. A preferred material is a synthetic resin membrane or film, higher in oxygen gas barrier properties, such as polyacrylonitrile nylon, polymethacrylonitrile, ethylene vinyl alcohol polymer, polyvinylidene chloride, polyethylene terephthalate or polyvinyl chloride. Other materials, on which are laminated the above-mentioned resin of higher oxygen gas barrier properties or metallic foil such as aluminium foil, may meet the requirements to fulfil the relevant purpose. The impervious sheet (11), the thickness of which depends on its material, needs to maintain a flat surface of the medium, when filling the Petri dish with the medium. This may be achieved by the strength of the sheet (11). In the case of a solid medium, the sheet (11) may be reinforced by insertion of a flat reinforcing member into a clearance between the impervious sheet (11) and the Petri dish lid (5) until the medium has solidified. Furthermore, if the small aperture (4) on the bottom (3) of the Petri dish is detachably equipped with a sealing lid (6), the preferred condition is realised, maintaining the aseptic state of the inside of the Petri dish proper (1) which has previously been sterilized. Thus the entire Petri dish can be kept in the aseptic state, similarly to the conventional Petri dish.

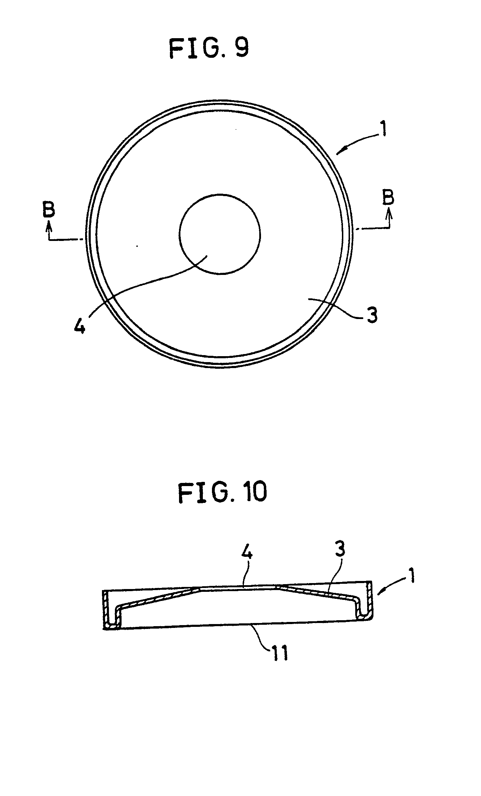

As shown in Figures 9 and 10, the bottom (3) of the Petri dish can be made such that the bottom (3) slopes from the periphery of the Petri dish proper (1) to the small aperture (4). In this case when pouring the media into the Petri dish, it is possible to fill the Petri dish with the media without giving rise to foam.

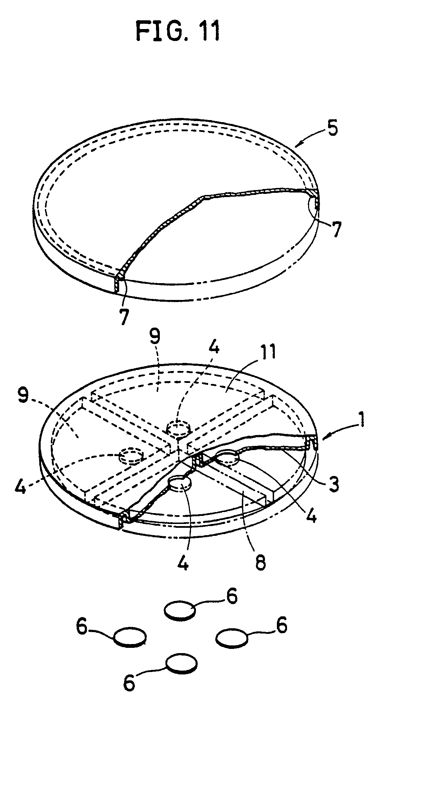

Figure 11 illustrates an embodiment wherein partitions (8) divide the Petri dish proper (1) into four sub-sections. Each of the sub-sections (9) is provided with a respective small aperture (4), whereby as the separate pouring of media into each sub-section (9) is completed, its small aperture (4) can be sealed by a sealing lid (6). This is advantageous in minimizing the risk of contamination by bacteria.

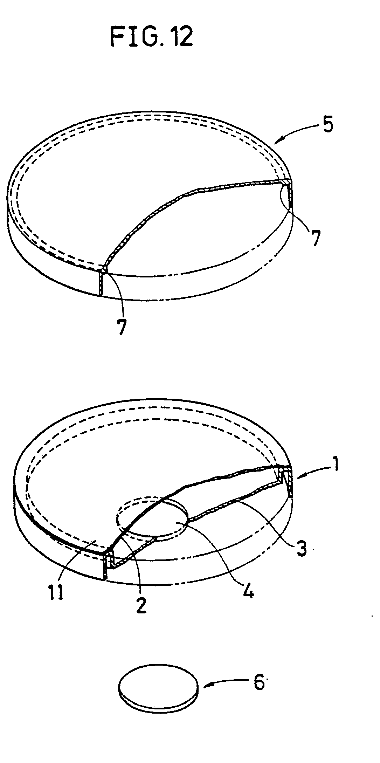

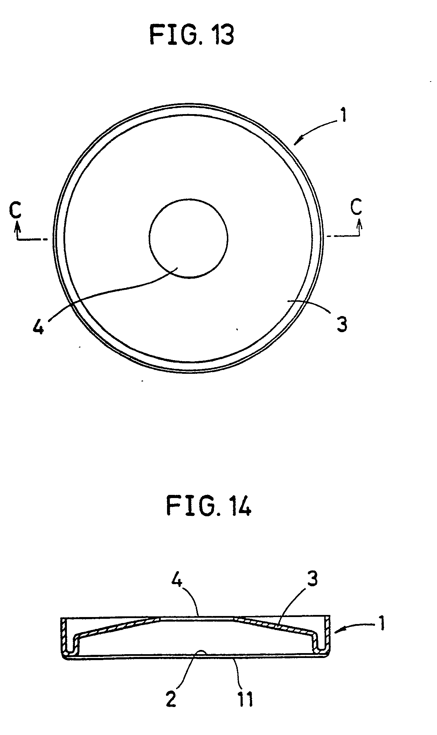

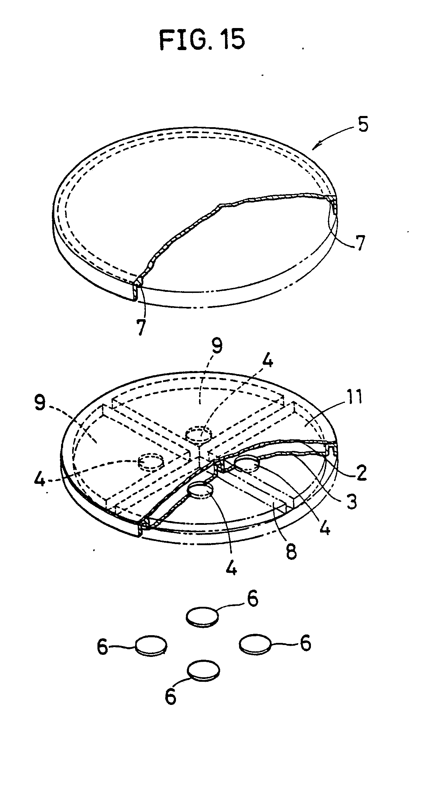

Figures 12 to 15 illustrate an embodiment using both the foregoing pervious sheet (2) and impervious sheet (11). The mouth at the upper edge of the Petri dish proper (1) is covered with the pervious sheet (2), which is in turn detachably covered with the impervious sheet (11).

The Petri dish for cultivating the bacteria according to the present invention can be utilized for all kinds of agar media, other than the liquid or semifluid media; although it is even possible to use these as mentioned below.

An embodiment of the method of inspecting drug susceptibility of bacteria according to the present invention will now be described as follows:

4

This embodiment uses a Petri dish such as shown in Figures 4 to 6, i.e., a Petri dish sub-divided into four, wherein the mouth at the upper edge of the Petri dish proper (1) is covered with the pervious sheet (2). The Petri dish proper (1) is provided with the partitions (8) the height of which matches the distance between the bottom (3) of the Petri dish and the pervious sheet (2). The bottom (3) of the Petri dish is provided with an aforesaid small aperture (4) which communicates with all the sub-sections (9) produced by the above-mentioned partitions (8). The small aperture (4) is equipped with a sealing lid (6).

As the above-mentioned pervious sheet (2), porous polyvinylidene fluoride membrane (bore: 0.45 μm) is used. Using Müller-Hinton agar medium as the medium, a fixed amount of diluted solution of each drug is added to the medium in order to obtain such a drug concentration as shown in List (1). One sub-section (9) of each Petri dish is left as a control, and media with respective different drug concentrations are poured separately into the three other sub-sections (9) and solidified therein. (Since this provides 3 media per Petri dish, 12 different media need four Petri dishes). Plain Müller-Hinton medium is separately poured into the sub-sections (9) used as controls.

First of all, a specimen of pus from a patient is manually inoculated on the pervious sheet (2) of an above-mentioned sub-divided Petri dish.

Next, the pus from the patient is inoculated by a different method, using a spiral plater (made by Spiral System Instruments, U.S.A). This spiral plater, which is an inoculating device incorporated into an automatic system for measuring the number of live bacteria, being developed and evaluated by FDA in U.S.A. performs the inoculation process in such a way that the material to be tested is spirally smeared on the medium, while the material to be tested is given a density gradient which is subject to the distance from the center of the medium.

For this reason, according to the method of the present invention, if the material to be tested is inoculated by being smeared on the pervious sheet (2) at the predetermined density gradient, a simple completion of measuring the number of isolated colonies per a fixed area will enable the total number of live bacteria contained in the unit quantity of the material to be tested to be counted out, and, furthermore, of course, in addition to the drug susceptibility value in accordance with the conventional standard, patterns of the sensitivity of the bacteria causing the disease to each drug at its various kinds of concentration stages to be easily and rapidly obtained.

As a result of using the above-mentioned spiral plater as the pathogenic bacteria in this specimen, *staphylococcus aureus* is isolated at the rate of $2.8 \times 10^4$ CFU/ml.

Using the conventional judgement standard based on the existence of growth, reference to List (1) enables a judgment to be made that MIC value of ABPC to the above-mentioned isolated bacteria is 1.56 μg/ml, that of CTM thereto is 0.78 μg/ml, those of CZX, GM, and NFLX thereto are 6.25, over 50, 3.13 μg/ml, respectively.

Paying attention to a change in the number of isolated colonies at the corresponding concentration of each drug may suggest that according to the conventional standard, MIC value of GM comes to be over 50 μg/ml, but actually, at 6.25 μg/ml of concentration, about 50% of bacteria to be tested shows the sensitivity.

| Pathogenic bacteria: *Staphylococcus aureus* Controlling medium: Müller-Hinton medium (2.8×10⁴ CFU/ml) | | | | | |
|---|---|---|---|---|---|
| Drug concentration (μg/ml) \ Name of drug | ABPC (CFU/ml) | CTM (CFU/ml) | CZX CFU/ml) | GM CFU/ml) | NFLX (CFU/ml) |
| 0.025 | $2.7 \times 10^4$ | $2.9 \times 10^4$ | $2.8 \times 10^4$ | $2.9 \times 10^4$ | $2.8 \times 10^4$ |
| 0.05 | $2.8 \times 10^4$ | $2.6 \times 10^4$ | $2.9 \times 10^4$ | $2.6 \times 10^4$ | $2.8 \times 10^4$ |
| 0.1 | $2.9 \times 10^4$ | $2.9 \times 10^4$ | $2.7 \times 10^4$ | $2.9 \times 10^4$ | $2.6 \times 10^4$ |
| 0.2 | $2.6 \times 10^4$ | $2.6 \times 10^4$ | $3.0 \times 10^4$ | $2.6 \times 10^4$ | $2.9 \times 10^4$ |
| 0.39 | $4.3 \times 10^3$ | $1.8 \times 10^4$ | $2.7 \times 10^4$ | $2.6 \times 10^4$ | $2.8 \times 10^4$ |
| 0.78 | $1.2 \times 10^2$ | 0 | $2.5 \times 10^4$ | $2.9 \times 10^4$ | $2.7 \times 10^4$ |
| 1.56 | 0 | 0 | $5.8 \times 10^3$ | $2.9 \times 10^4$ | $3.5 \times 10^3$ |
| 3.13 | 0 | 0 | $3.6 \times 10^2$ | $2.6 \times 10^4$ | 0 |
| 6.25 | 0 | 0 | 0 | $1.7 \times 10^4$ | 0 |
| 12.5 | 0 | 0 | 0 | $8.0 \times 10^3$ | 0 |
| 25 | 0 | 0 | 0 | $6.8 \times 10^3$ | 0 |
| 50 | 0 | 0 | 0 | $6.2 \times 10^3$ | 0 |

ABPC: ampicillin
CTM: cefotiam
CZX: ceftizoxime
GM; gentamicin
NFLX: norfloxacin

The above described constructions of Petri dish for cultivating bacteria, embodying the present invention, all make it possible to fill the Petri dish proper (1) with the medium without opening the Petri dish lid (5). The manner of filling the dish is that, while the Petri dish lid (5) is in place, the Petri dish is turned upside down, and the medium or media are poured in, separately from each other if more than one, through the small aperture (4) in the bottom (3) of the Petri dish. For this reason, since the Petri dish lid (5) is not opened when pouring the media, the danger of the media being contaminated by various bacteria is reduced in comparison with the conventional type of Petri dish.

When the pervious sheet (2) is used, the media is solidified on the previous sheet (2) which is a bottom surface while the dish is upside down. The pervious sheet (2) acts as the cultivation surface when the media are subsequently used for inoculation and cultivation. Solidification of the medium does not need a flat table as is needed conventionally, because vibration during the solidification does not cause turbulence on the eventual working surface of the medium. When using the impervious sheet (11), the impervious sheet (11) is removed when the medium is to be used for inoculation and cultivation and the foregoing effect may be also produced.

In the case of the Petri dish which is partitioned into sub-sections according to embodiments of the present invention, the level of the cultivation surface of the media with which each fraction is filled is unified by the aforementioned pervious sheet (2) or impervious sheet (11). Inoculation by an automatic inoculating device such as the spiral plater—which has difficulty in operating with a conventional partitioned Petri dish due to the difference in level between the media and the partitions (8)—becomes possible. Also in the case of manual inoculation, it is possible to inoculate bacteria into a majority of media in one operation.

Furthermore, when the Petri dish with the aforementioned pervious sheet is used, since the shape of the medium with which the Petri dish proper (1) is filled is restricted by the Petri dish proper (1) and the pervious sheet (2), it is possible to fill the dish with a liquid medium, provided that a resin of higher water absorbency comprising such materials as polyacrylic soda, starch polyacrylate or PVA, or a solidifying agent comprising such materials as alginic acid of natural origin or carboxy methyl cellulose, are sealed in the Petri dish proper (1). Thereby it is made easy to carry out a preparation of the medium containing the drug, which conventionally needed complicated operations. In addition, if each of the sub-sections (9) of the Petri dish proper (1) is filled with media which differ from each other in the concentration of the drug which they contain the resulting arrangement is convenient for carrying out a drug susceptibility test.

In the case of the Petri dish using the impervious sheet (11), the media are sealed between the impervious sheet (11) and the Petri dish proper (1) during storage or in transit after filling the Petri dish with the media. Thereby the media do not come into contact with the air, consequently preventing their drying and deterioration due to oxidation, wetting of the cultivation surface due to humidity, and contamination by various bacteria, during storage. This possibility that the media poured into the inside are stored in a state where they are screened from the air can be utilised when cultivating *anaerobic bacteria*. If the media are deaerated in advance by pressure reduction, boiling, ultrasonic treatment, etc. before being poured into the dish, storage while screened from the air provides a medium which has less dissolved oxygen than a conventional medium.

Incidentally, the petri dish for cultivating the bacteria according to the present invention can also be used for the screening of pathogenic bacteria, using several individual dishes with appropriate media on their sub-divisions.

In the method of the present invention, utilization of the pervious sheet (2) as the cultivation surface enables an extremely flat cultivation surface to be obtained in spite of employing a Petri dish which is partitioned into subsections. In addition to making the manual inoculation of bacteria easier, utilization of such an automatic inoculating device as the spiral plater becomes possible.

## Claims

1. A Petri dish for cultivating bacteria characterised in that the mouth at the upper edge of the Petri dish proper is covered with at least one sheet and at least one small aperture for filling the dish is provided in the bottom of said Petri dish.

2. A Petri dish as defined in Claim 1, wherein said sheet is pervious to a component of a medium or media with which the inside of said Petri dish proper is filled.

3. A Petri dish as defined in Claim 1, wherein said sheet is impervious to a component of a medium or media with which the inside of said Petri dish proper is filled, the sheet detachably covering said Petri dish.

4. A Petri dish as defined in Claim 2, wherein said sheet is detachably covered with another sheet which is impervious to the component of the medium or media with which the inside of said Petri dish is filled.

5. A Petri dish as defined in Claim 2 or Claim 4, wherein the pervious sheet can hold bacteria on the surface thereof.

6. A Petri dish as defined in Claim 3, wherein said sheet is impervious to water and oxygen.

7. A Petri dish as defined in Claim 4, wherein said other sheet is impervious to water and oxygen.

8. A Petri dish as defined in any of Claims 1 to 7, wherein said Petri dish proper is sub-divided by partitions the height of which extends between the bottom of said Petri dish and said sheet.

9. A Petri dish as defined in any of Claims 1 to 8, wherein said small aperture is provided with a detachable sealing lid.

10. A method of testing drug susceptibility of bacteria characterised in that bacteria are inoculated into a media-filled Petri dish according to Claim 8.

11. A method as defined in Claim 10 wherein the Petri dish has a said sheet which is pervious to a component of the medium or media, which sheet is used as a cultivation surface.

12. A method as defined in Claim 10 or Claim 11 wherein the media in the sub-sections of the Petri dish differ in concentration of a drug contained in the media.

13. A method as defined in Claim 10 or Claim 11 wherein the media in the sub-sections of the Petri dish differ in the nature of drugs contained in them.

14. A method as defined in any of Claims 10 to 13 wherein said bacteria inoculation is a spiral inoculation which is performed, while forming a density gradient.

## Patentansprüche

1. Petrischale zur Züchtung von Bakterien, dadurch gekennzeichnet, daß die Öffnung am oberen Rand der eigentlichen Petrischale mit mindestens einer Schicht abgedeckt ist und im Boden der Petrischale mindestens eine kleine Öffnung zum Füllen der Schale vorgesehen ist.

2. Petrischale nach Anspruch 1, dadurch gekennzeichnet, daß die Schicht für eine Komponente eines Mediums oder von Medien, mit welchem das Innere der eigentlichen Petrischale gefüllt ist, durchlässig ist.

3. Petrischale nach Anspruch 1, dadurch gekennzeichnet, daß die Schicht für eine Komponente eines Mediums oder von Medien, mit welchem das Innere der eigentlichen Petrischale gefüllt ist, undurchlässig ist, und daß die Schicht die Petrischale abnehmbar abdeckt.

4. Petrischale nach Anspruch 2, dadurch gekennzeichnet, daß die Schicht abnehmbar mit einer anderen Schicht bedeckt ist, die undurchlässig ist für die Komponente des Mediums oder der Medien, mit welchem das Innere der Petrischale gefüllt ist.

5. Petrischale nach Anspruch 2 oder 4, dadurch gekennzeichnet, daß die durchlässige Schicht auf ihrer Oberfläche Bakterien halten kann.

6. Petrischale nach Anspruch 3, dadurch gekennzeichnet, daß die Schicht undurchlässig für Wasser und Sauerstoff ist.

7. Petrischale nach Anspruch 4, dadurch gekennzeichnet, daß die andere Schicht undurchlässig für Wasser und Sauerstoff ist.

8. Petrischale nach einem der Ansprüche 1 bis 7, dadurch gekennzeichnet, daß die eigentliche Petrischale durch Wandungen unterteilt ist, die sich in der Höhe zwischen dem Boden der Petrischale und der Schicht erstrecken.

9. Petrischale nach einem der Ansprüche 1 bis 8, dadurch gekennzeichnet, daß die kleine Öffnung mit einem entfernbaren Verschlußdeckel versehen ist.

10. Verfahren zur Prüfung der Drogenempfindlichkeit von Bakterien, dadurch gekennzeichnet, daß die Bakterien in eine mit Medien gefüllte Petrischale nach Anspruch 8, eingeimpft sind.

11. Verfahren nach Anspruch 10, dadurch gekennzeichnet, daß die Petrischale mit einer für eine Komponente des Mediums oder der Medien durchlässigen Schicht versehen ist, die als Kulturoberfläche verwendet wird.

12. Verfahren nach Anspruch 10 oder 11, dadurch gekennzeichnet, daß die Medien in den Unterteilungen der Petrischale sich in der Konzentration einer Droge unterscheiden, die in den Medien enthalten ist.

13. Verfahren nach Anspruch 10 oder 11, dadurch gekennzeichnet, daß die Medien in den Untergliederungen der Petrischale sich in der Art der in ihnen enthaltenen Drogen unterscheiden.

14. Verfahren nach einem der Ansprüche 10 bis 13, dadurch gekennzeichnet, daß die Bakterienimpfung in Spiralform erfolgt, die unter Bildung eines Dichtegradienten durchgeführt wird.

**Revendications**

1. Une boîte de Petri pour la culture de bactéries, caractérisée par le fait que l'ouverture située au niveau du bord supérieur de la boîte de Petri proprement dite est couverte par au moins une feuille, et au moins un petit orifice servant à remplir la boîte est ménagé dans le fond de ladite boîte de Petri.

2. Une boîte de Petri telle que définie dans la revendication 1, dans laquelle ladite feuille est perméable à un composant d'un milieu ou de milieux avec lesquels est rempli l'intérieur de ladite boîte de Petri proprement dite.

3. Une boîte de Petri telle que définie dans la revendication 1, dans laquelle ladite feuille est imperméable à un composant d'un milieu ou de milieux avec lesquels est rempli l'intérieur de ladite boîte de Petri proprement dite, la feuille couvrant de façon détachable ladite boîte de Petri.

4. Une boîte de Petri telle que définie dans la revendication 2, dans laquelle ladite feuille est couverte de façon détachable par une autre feuille qui est imperméable au composant du milieu ou des milieux avec lesquels est rempli l'intérieur de ladite boîte de Petri.

5. Une boîte de Petri telle que définie dans la revendication 2 ou la revendication 4, dans laquelle la feuille perméable peut supporter des bactéries sur sa surface.

6. Une boîte de Petri telle que définie dans la revendication 3, dans laquelle ladite feuille est imperméable à l'eau et à l'oxygène.

7. Une boîte de Petri telle que définie dans la revendication 4, dans laquelle ladite autre feuille est imperméable à l'eau et à l'oxygène.

8. Une boîte de Petri telle que définie dans l'une quelconque des revendications 1 à 7, dans laquelle ladite boîte de Petri proprement dite est compartimentée par des cloisons dont la hauteur s'étend entre le fond de ladite boîte de Petri et ladite feuille.

9. Une boîte de Petri telle que définie dans l'une quelconque des revendications 1 à 8, dans laquelle ledit petit orifice est équipé d'un couvercle d'obturation amovible.

10. Un procédé pour ètudier la sensibilité de bactéries à un ou plusieurs médicaments, caractérisé par le fait que les bactéries sont inoculées dans une boîte de Petri selon la revendication 8 remplie avec des milieux.

11. Un procédé tel que défini dans la revendication 10, dans lequel la boîte de Petri comporte une feuille que est perméable à un composant du milieu ou des milieux, cette feuille étant utilisée comme surface de culture.

12. Un procédé tel que défini dans la revendication 10 ou la revendication 11, dans lequel les milieux présents dans les compartiments de la boîte de Petri différent par la concentration d'un médicament contenu dans les milieux.

13. Un procédé tel que défini dans la revendication 10 ou la revendication 11, dans lequel les milieux présents dans les compartiments de la boîte de Petri différent par la nature de médicaments qu'ils contiennent.

14. Un procédé tel que défini dans l'une quelconque des revendications 10 à 13, dans lequel ladite inoculation de bactéries est une inoculation en spirale qui est exécutée tout en formant un gradient de densité.

# FIG.1

# FIG. 2

5

7

# FIG. 3

1

3

4

# FIG.4

# FIG. 5

# FIG. 6

# FIG.7

1

A A

3

4

# FIG.8

4 3

1

11

## FIG. 9

## FIG. 10

# FIG. 11

# FIG. 12

# FIG. 13

# FIG. 14

## FIG. 15